# EUROPEAN PATENT SPECIFICATION

(11) **EP 3 742 258 B1**
(45) Date of publication and mention of the grant of the patent: **31.05.2023**
(21) Application number: 18900802.2
(22) Date of filing: 04.12.2018
(51) Int. Cl.: G06F 1/16, G06F 3/01, A61H 1/00, A61B 5/00, A61B 5/024, A61F 7/00, A61H 23/02

(54) **WEARABLE DEVICE AND MOUNTING FIXTURE**
AM KÖRPER TRAGBARE VORRICHTUNG UND MONTAGEVORRICHTUNG
DISPOSITIF VESTIMENTAIRE ET SUPPORT DE MONTAGE

(30) Priority: 19.01.2018 JP 2018007113
(43) Date of publication of application: 25.11.2020
(73) Proprietor: Sony Group Corporation, Tokyo 108-0075 (JP)
(72) Inventor: KAWAGUCHI, Hiroto, Tokyo 108-0075 (JP); MIZUTA, Daisuke, Tokyo 108-0075 (JP)
(74) Representative: D Young & Co LLP
(86) International application number: PCT/JP2018/044518
(87) International publication number: WO 2019/142525

(56) References cited:
- WO-A1-2016/175052
- WO-A1-2016/175052
- JP-A- 2015 167 611
- JP-A- 2016 197 349
- JP-A- 2016 197 349
- US-B1- 9 711 060

## Description

### Technical Field

The present technology relates to a wearable apparatus and a wearing part used therefor capable of being worn, for example, by a user's finger or wrist.

### Background Art

In recent years, development of a haptic feedback apparatus capable of being worn by a user's finger, wrist, or the like and an apparatus for detecting biodata such as a blood flow and a pulse has been progressed in a variety of fields. Such wearable apparatus includes a wearing part, and an electronic device such as a haptic feedback device and a sensor device mounted to the wearing part (for example, see Patent Literature 1).

The wearing part is roughly classified into a flexible type formed of a soft cloth, synthetic leather, etc. and a rigid type in which rigid individual parts are combined. The rigid type ensures mobility upon wearing, for example, by providing a degree of rotation freedom among the individual parts.

### Citation List

### Patent Literature

Patent Literature 1: Japanese Patent Application Laid-open No. 2016-51319

### Disclosure of Invention

### Technical Problem

In this kind of the wearable apparatus, it needs to ensure a stable fitting property of the wearing part with respect to an object on which it is to be worn such as a human body in order to realize haptic feedback presentation with a desirable vibration force or stable acquisition of the biodata.

However, the above-described wearing part has a problem that it is difficult to accommodate the size variations of the objects on which it is to be worn. For example, as to vibration haptics, haptics may be weak as compared to a gripping device such as a smartphone. This is because vibration from a vibration device is difficult to be transmitted to a skin since the wearing part is not firmly in intimate contact with the finger, etc., or softness of the wearing part weakens the vibration. On the other hand, in the rigid type wearing part, if a size of the finger is not matched, a wearing position may be misaligned, or fastening is too strong, causing a feeling of pain. Some may allow a size adjustment by using a buckle or a hook-and-loop fastener, but there is also a problem that wearing and size adjustment works are troublesome.

The present technology is made in view of the above-mentioned circumstances, and it is an object of the present technology to provide a wearable apparatus and a wearing part that ensure a stable fitting property not depending on a size of an object on which the wearing part is to be worn. Prior art includes: JP 2016 197349 A and WO 2016/175052, which each disclose a prior art device. Solution to Problem

Aspects of the present invention are defined by the appended claims.

### Advantageous Effects of Invention

As described above, according to the present invention, it can ensure a stable fitting property not depending on a size of the object on which the wearing part is to be worn.

It should be noted that the effects described here are not necessarily limitative and may be any of effects described in the present disclosure.

### Brief Description of Drawings

[Fig. 1] Fig. 1 is an overall perspective view of a wearable apparatus according to a first embodiment of the present invention.
[Fig. 2] Fig. 2 is a perspective view showing a status that the wearable apparatus is worn by a user.
[Fig. 3] Fig. 3 is a front view of the wearing part of the wearable apparatus.
[Fig. 4] Fig. 4 is a front view schematically showing a deforming process of the wearing part.
[Fig. 5] Fig. 5 is a front view showing details of the wearing part.
[Fig. 6] Fig. 6 is front views of main parts of the wearing parts and shows modifications of shapes of bending parts.
[Fig. 7] Fig. 7 is front views of the wearing parts showing arrangement examples of electronic devices.
[Fig. 8] Fig. 8 is conceptual views for illustrating actions of the wearable apparatuses.
[Fig. 9] Fig. 9 is a front view of a seal ring according to a comparative embodiment.
[Fig. 10] Fig. 10 is a front view showing evaluation conditions of the wearing part.
[Fig. 11] Fig. 11 is an experimental result showing actions of the wearing parts including the comparative embodiment.
[Fig. 12] Fig. 12 is a simulated result showing a stress distribution of the wearing part.
[Fig. 13] Fig. 13 is a front view of a wearing part according to a second embodiment of the present invention.
[Fig. 14] Fig. 14 is a side view of the wearing part.
[Fig. 15] Fig. 15 is a perspective view of a wearing part according to a third embodiment of the present invention.
[Fig. 16] Fig. 16 is perspective views of a wearing part according to a fourth embodiment of the present invention.
[Fig. 17] Fig. 17 is a perspective view showing a wearable apparatus according to a fifth embodiment of the present invention.
[Fig. 18] Fig. 18 is a schematic front view showing structure modification of the wearing part.
[Fig. 19] Fig. 19 is schematic front views showing form modifications of the wearing part.

### Mode(s) for Carrying Out the Invention

Hereinafter, embodiments of the present invention will be described with reference to the drawings.

### <First embodiment>

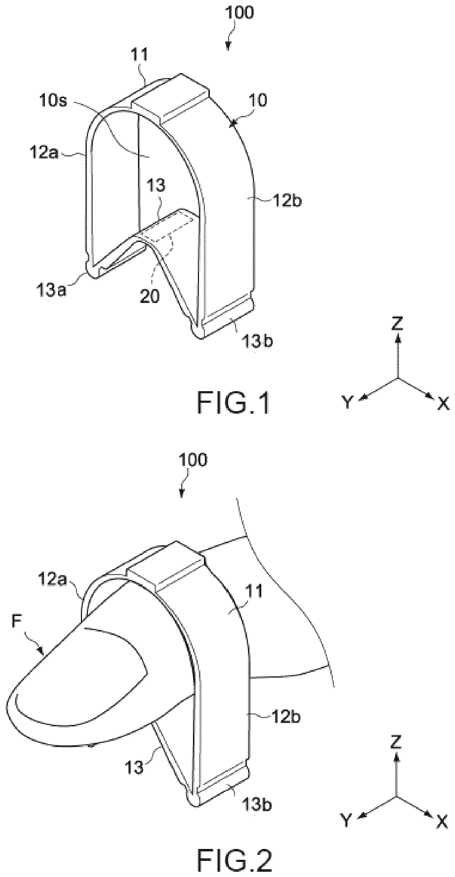

Fig. 1 is an overall perspective view of a wearable apparatus according to a first embodiment of the present invention, and Fig. 2 is a perspective view showing a status that the wearable apparatus is worn by a user.

In Figs. 1 and 2, the X, Y, and Z axes show three axes directions orthogonal to each other.

### [Wearable apparatus]

A wearable apparatus 100 in this embodiment is a ring side device capable of being worn by a user's finger F. The finger F is not especially limited and is typically an index finger (a second finger). A wearing position of the finger F is not especially limited and may be a distal interphalangeal joint or a proximal interphalangeal joint or therebetween.

The wearable apparatus 100 includes a wearing part 10 and an electronic device 20.

### (Wearing part)

The wearing part 10 has an annular member including an insertable hollow part 10s into which the finger F as an object on which the wearing part is to be worn is capable of being inserted, as shown in Fig. 1. The wearing part 10 has a spring property as a whole and is capable of enlarging an opening area of the hollow part 10s within a predetermined range from a natural status as shown. The size of the hollow part 10s is set to a predetermined size such that a maximum diameter is smaller than a maximum diameter of the index finger of the user.

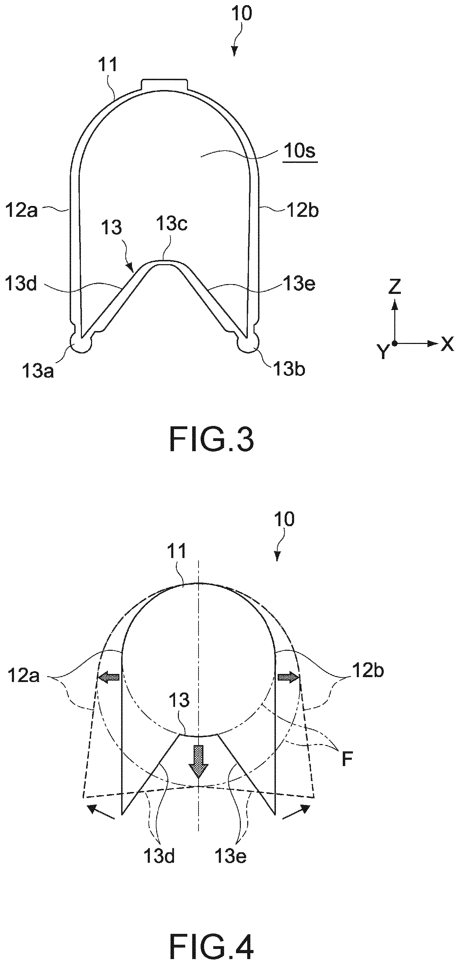

Fig. 3 is a front view of the wearing part 10 viewed in the Y axis direction.

As shown in Fig. 3, the wearing part 10 is formed in a non-convex polygonal shape. The wearing part 10 includes a base part 11, a pair of arms 12a and 12b, and a pressing part 13, and has an integrated structure in which they are continuously connected. The base part 11, the arms 12a and 12b, and the pressing part 13 are each formed with the same width.

The base part 11 has an arch shape, and its inner perimeter surface is capable of being in contact with an upper surface of the finger F inserted into the hollow part 10s, as shown in Fig. 2.

The pair of arms 12a and 12b are linked to both ends of the base part 11 in a perimeter direction. The pair of arms 12a and 12b extend along in the Z axis direction and are each formed linearly having the same length. Note that the arms 12a and 12b may extend so as to incline inward or outward in the Z axis direction.

The pressing part 13 is linked to edges of the pair of arms 12a and 12b and is opposed to the base part 11 across the hollow part 10s. The pressing part 13 has a convex shape at an inner perimeter side and is bending-deformable.

The pressing part 13 has a pair of bending parts 13a and 13b linked to the pair of arms 12a and 12b and a top part 13c positioned between the pair of bending parts 13a and 13b. Angles of the bending parts 13a and 13b of the pressing part 13 are increased depending on a distance increase between the pair of arms 12a and 12b. Thus, the bending parts 13a and 13b are elastically deformable in a direction in which the top part 13c is apart from the base part 11.

The top part 13c has a plane shape and its inner perimeter surface is capable of being in contact with a lower surface of the finger F inserted into the hollow part 10s, as shown in Fig. 2. The shape of the top part 13c is not limited thereto, and may be a curved shape or an edge shape having a ridgeline.

The pressing part 13 further includes a pair of linking parts 13d and 13e that link between the pair of bending parts 13a and 13b and the top part 13c. The pair of linking parts 13d and 13e extend linearly from the both ends of the top part 13c toward the bending parts 13a and 13b. An angle between the pair of linking parts 13d and 13e is variable depending on a distance change between the pair of arms 12a and 12b.

Fig. 4 is a front view schematically showing a deforming process of the wearing part 10.

As shown in Fig. 4, depending on a deformation amount of the pair of arms 12a and 12b around an axis (radially outward) in parallel with the Y axis direction using the base part 11 as a fulcrum, an angle between the both linking parts 13d and 13e of the pressing part 13 is increased. The pressing part 13 is deformable from an inward convex shape in a natural status to a substantially flat shape or an outward convex shape.

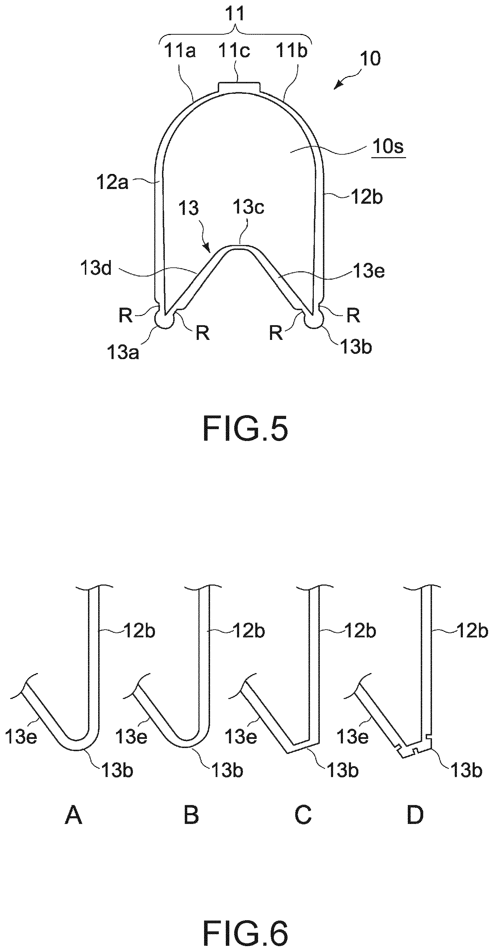

In order to achieve a diameter expansion action of the wearing part 10 as described above, a relationship among shapes and thicknesses of the respective parts of the wearing part 10 is favorably optimized. In this embodiment, as shown in Fig. 5, there are a pair of low rigid parts 11a and 11b linked to the pair of arms 12a and 12b and a high rigid part 11c positioned between the pair of low rigid parts 11a and 11b.

The high rigid part 11c is positioned at almost a center of the base part 11 and has rigidity higher than the pair of low rigid parts 11a and 11b. Specifically, the high rigid part 11c is formed thicker than the pair of low rigid parts 11a and 11b.

The pair of arms 12a and 12b are formed thicker than the pair of low rigid parts 11a and 11b and have rigidity higher than the pair of low rigid parts 11a and 11b. Accordingly, in a case where the pair of arms 12a and 12b are deformed radially outward, the pair of low rigid parts 11a and 11b are preferentially deformed, which makes the distance between the pair of arms 12a and 12b variable.

Similarly, the top part 13c of the pressing part 13 is formed thinner than the pair of linking parts 13d and 13e and has rigidity lower than the linking parts 13d and 13e. Accordingly, in a case where the pair of arms 12a and 12b are deformed radially outward, the pair of linking parts 13d and 13e are easily deformed to the top part 13c. Thus, the top part 13c is easily moved radially outward following the deformation of the arms 12a and 12b.

Furthermore, similarly, as thinner parts R are arranged on the pair of bending parts 13a and 13b at boundaries between the arms 12a and 12b and the linking parts 13d and 13e, the arms 12a and 12b and the linking parts 13d and 13e are easily deformed to the bending parts 13a and 13b. As a result, the above-described action effects are more easily provided.

Forms of the bending parts 13a and 13b are not limited to the above embodiments and a variety of forms shown, for example, in Figs. 6A to D may be utilized. By adding a shape effect of decreasing the thickness or the width, etc., power needed for changing the angles of the bending parts 13a and 13b can be decreased.

Note that one bending part 13b is illustrated in Figs. 6A to D and the same is applicable to the other bending part 13a.

As shown in Figs. 6A and B, the bending parts 13a and 13b may be formed in curved shapes. In this way, the bending parts 13a and 13b are more easily deformed. In this case, as shown in Fig. 6B, the bending parts 13a and 13b may be formed thinner than the arms 12a and 12b and the linking parts 13d and 13e.

On the other hand, the bending parts 13a and 13b may be multiply bent as shown in Figs. 6C and D. With such shape, the action effects may be provided similar to the above. In this case, thin parts (constrictions) may be arranged at appropriate positions of the bending parts 13a and 13b as shown in Fig. 6D so as to have rigidity lower than the arms 12a and 12b and the linking parts 13d and 13e.

The wearing part 10 is formed of, for example, a molded body of a synthetic resin material. The rigidity or elastic modulus of the wearing part 10 can be adjusted by a Young's modulus or a cross-sectional shape of a component material, which are optimized depending on a specification or an application of the wearing part 10.

The synthetic resin material is not especially limited, includes a synthetic rubber-based resin material such as silicone rubber and EPDM (ethylene propylene diene rubber), and is formed in a desirable shape by an appropriate forming method such as thermoforming and injection molding. The wearing part 10 may be formed by blow-molding a thermoplastic resin material such as PET (polyethylene terephthalate). Since the wearing part 10 is formed of the synthetic resin material, it is possible to utilize expansion and contraction of the material itself.

The wearing part 10 may be formed of a metal material. Examples of the metal material include an aluminum alloy, stainless steel, a copper alloy, and the like. The metal material is formed in an annular shape with the thickness so as to provide adequate elasticity (springiness). A forming method is not especially limited and an appropriate method including die casting, press work, welding, and the like may be utilized.

The wearing part 10 may be formed of two or more materials as described later. For example, the above-described resin material and the metal material may be combined, and a fiber material such as acrylic fiber, an elastic or non-elastic cloth material, a leather material such as synthetic leather and cowhide, or a cushioning material (rubber material) such as polyurethane may be arranged at an appropriate position.

### (Electronic device)

An electronic device 20 includes a haptic feedback device capable of presenting vibration haptics. The haptic feedback device typically includes a piezoelectric element, a vibration motor, a linear vibrator, and the like, and presents haptics to the finger F inserted into the hollow part 10s by inputting an electrical signal from the outside. In this case, the wearable apparatus 100 is an information output apparatus that, for example, guides directions, operates a machine such as a video game, or presents haptic feedback to the user when the user touches an image displayed using a virtual reality technology.

Alternatively, the wearable apparatus 100 may be medical equipment such as high frequency therapy equipment and low frequency therapy equipment. The haptic feedback device may be a compression device that presents a compression stimulus to a skin. In this case, the wearable apparatus 100 is a massager that applies a press pressure. The electronic device 20 may be a hot/cold presentation device that builds in a heater or a Peltier element.

On the other hand, the electronic device 20 may be a sensor device including an inertial sensor that detects acceleration or an angular velocity, a biosensor that detects biodata such as a blood flow, a temperature and perspiration, and a myoelectric sensor that detects a weak electrical signal of a human body. In this case, the wearable apparatus 100 is an information input apparatus that operates a pointer, inputs a gesture, or acquires the biodata from a patient.

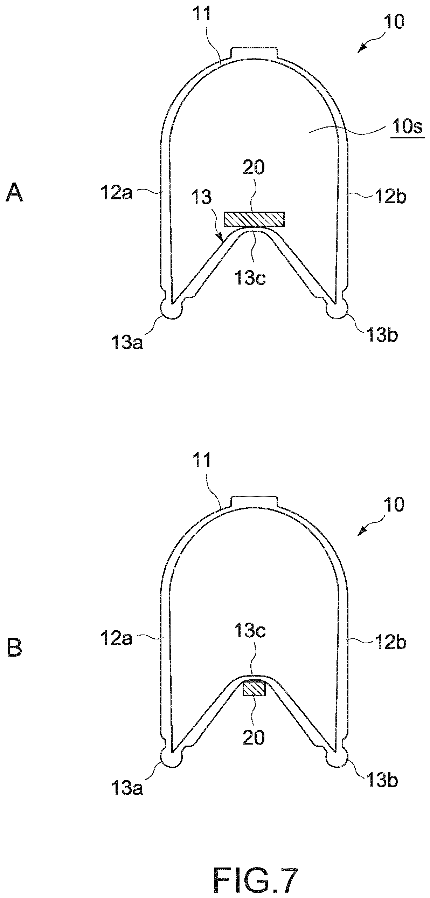

The electronic device 20 is desirably arranged at a position in which deformation of each part of the wearing part 10 is not inhibited. The electronic device 20 is typically arranged at the top part 13c of the pressing part 13 but may be arranged at the base part 11. The electronic device 20 may be arranged at an inner surface of the top part 13c as shown in Fig. 7A or may be arranged at an outer surface of the top part 13c as shown in Fig. 7B. The electronic device 20 is fixed to the top part 13c by using an appropriate joining material such as an adhesive and a bond. Alternatively, the electronic device 20 may be buried inside of the top part 13c (see Fig. 1).

The size of the electronic device 20 is not especially limited and may be appropriately set depending on a type of the device or an attachment position to the pressing part 13. As shown in Figs. 7A and B, in a case where the electronic device 20 is attached to the inner surface of the top part 13c, a larger device may be used as compared with the case that the electronic device 20 is attached to the outer surface of the top part 13c.

The electronic device 20 is arranged at the pressing part 13 (or base part 11) of the wearing part 10 and can thus transmit the vibration to the finger F without effect on a shape change of the wearing part 10 or can acquire information from the finger F. In addition, since the pressing part 13 (or base part 11) is less subject to the shape change, a contact between the electronic device 20 and the finger F can be stably maintained.

### [Action of wearable apparatus]

In the wearable apparatus 100 according to this embodiment configured as described above, a distance between the pair of arms 12a and 12b is changed to meet the size of the finger F inserted into the hollow part 10s. Furthermore, a relative distance between the base part 11 and the pressing part 13 to meet the distance change between the pair of arms 12a and 12b.

In other words, the pair of arms 12a and 12b function as a spreader spring that deforms to spread in a width direction depending on the size of the finger, and the pressing part 13 functions as a thickness adjustment spring that deforms to spread in a thickness direction depending on the size of the finger.

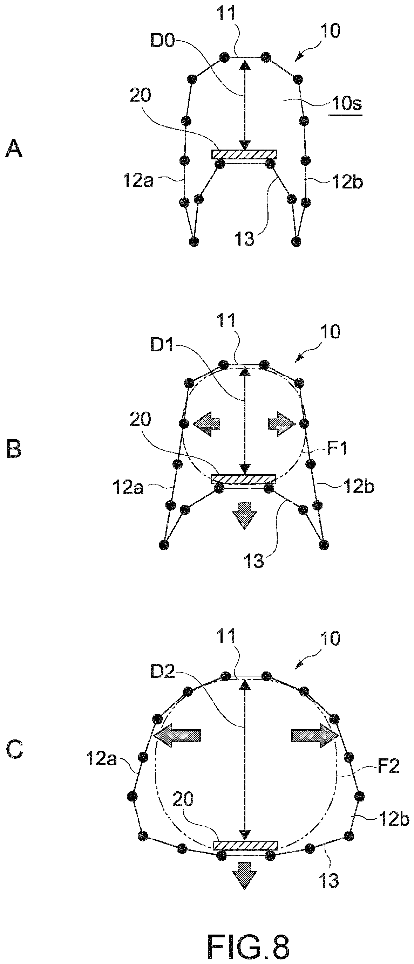

Fig. 8A to C are conceptual diagrams for explaining a diameter expansion action of the wearable apparatus 100.

Fig. 8A shows a natural status of the wearing part 10, in which the hollow part 10s having the predetermined size is partitioned by the base part 11, the pair of arms 12a and 12b, and the pressing part 13. Once a finger F1 having a size larger than the predetermined size into the hollow part 10s, the pair of arms 12a and 12b are deformed radially outward as shown by arrows in Fig. 8B such that the hollow part 10s spreads to the size capable of surrounding the finger F1.

On the other hand, if a finger F2 thicker than the finger F1 is inserted into the hollow part 10s, the pair of arms 12a and 12b and the pressing part 13 are further deformed radially outward as shown by arrows in Fig. 8C, the hollow part 10s spreads to the size capable of surrounding the finger F2.

Any of the pair of arms 12a and 12b and the pressing part 13 has the spring property. Thus, the wearing part 10 is deformed depending on the size of the finger F1 or F2 inserted into the hollow part 10s insert, pinches the finger F1 or F2 in the thickness direction between the base part 11 and the pressing part 13 (electronic device 20), and pinches the finger F1 or F2 in the width direction between the pair of arms 12a and 12b.

Note that when the wearing part 10 is removed from the finger F1 or F2, the wearing part 10 autonomously returns to the original natural status.

As the fingers F1 and F2, different user's fingers of, e.g., a male, a female, an adult, a child, etc. are assumed. A width of a proximal joint of a second finger is 14.1 mm to 21.5 mm (male average value is 18.0 mm, and female average value is 15.9 mm), and a thickness of the proximal joint of the second finger is 13.4 mm to 20.1 mm (male average value is 16.8 mm, and female average value is 14.8 mm) (as above, see https://www.dh.aist.go.jp/database/hand/data/all.pdf).

Depending on the user's fingers F1 or F2 with such different size, respective moving amounts of the pair of arms 12a and 12b and the pressing part 13 in the radially outward direction are determined. Depending on the determined moving amounts, the distance between the base part 11 and the electronic device 20 is increased from the natural status D0 to D1 or D2. Depending on an increment thereof, a pressing force of the electronic device 20 with respect to the finger F1 or F2 is determined.

Thus, according to the embodiment, the fitting property of the wearing part 10 is stably ensured with respect to each user to be assumed without being influenced by size variations of the user's fingers F. In addition, since the electronic device 20 can stably bring in contact with the finger F not depending on the size of the finger F, it becomes possible to transmit or acquire desired information.

Advantageously, a counterforce caused by a finger size difference can be decreased according to this embodiment as compared with a circle ring-like elastic member such as a seal ring.

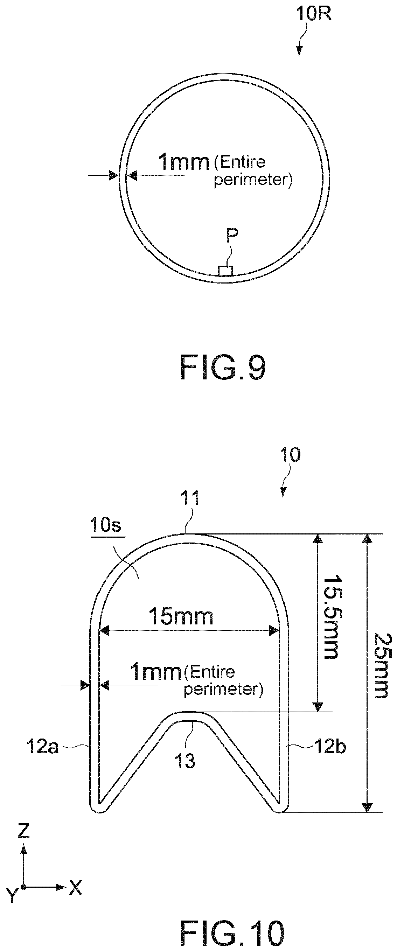

The present inventors prepared a seal ring 10R having the shape shown in Fig. 9 according to a comparative embodiment and evaluated a counterforce difference from the wearing part 10 of this embodiment.

The seal ring 10R according to the comparative embodiment had an inner diameter of 15 mm and a thickness of 1 mm and formed of a rubber material having the Young's modulus of 1 MPa and a Poisson's ratio of 0.49. A protrusion P having a width of 1 mm and a height of 0.5 mm was formed toward the center at a part of an inner perimeter of the seal ring 10R.

On the other hand, as shown in Fig. 10, the wearing part 10 of this embodiment had a total length in the Z axis direction of 25 mm, an opposed distance between inner surfaces of the pair of arms 12a and 12b of 15 mm, a distance between the top part outer surface of the base part 11 and the inner surface of the pressing part 13 in the Z axis direction of 15.5 mm, and the thickness of 1 mm around an entire perimeter, and was formed of a silicone rubber material.

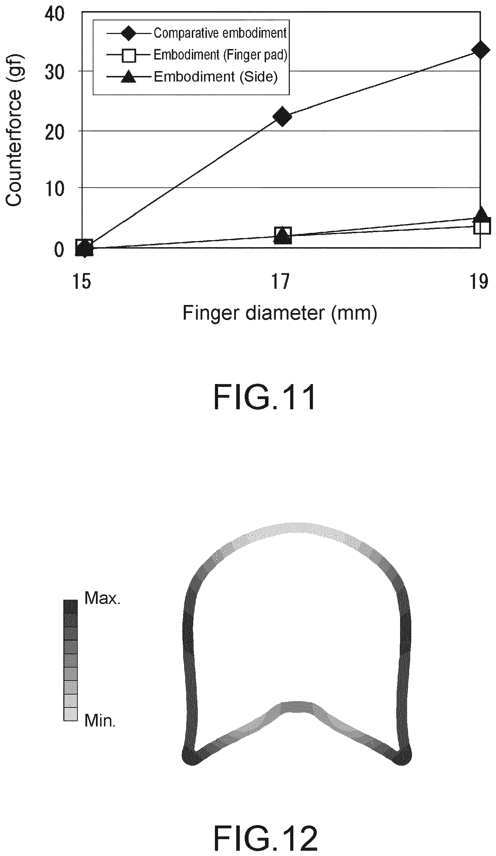

Objects to be inserted having diameters of 15 mm, 17 mm, and 19 mm were inserted into the seal ring 10R according to the comparative embodiment and the counterforce acted on the protrusion P was evaluated. As a result, the counterforce of the object to be inserted having the diameter of 15 mm was 0.589 [gf], the counterforce of the object to be worn having the diameter of 17 mm was 22.58 [gf], and the counterforce of the object to be worn having the diameter of 19 mm was 33.90 [gf], as shown in Fig. 11.

On the other hand, the objects to be worn having diameters of 15 mm, 17 mm, and 19 mm were inserted into the hollow part 10s of the wearing part 10 according to this embodiment shown in Fig. 8 and the counterforce in the X axis direction (counterforce acted on arms 12a and 12b) and the counterforce in the Z axis direction (counterforce acted on pressing part 13) were evaluated, respectively.

As a result, as shown in Fig. 11, the counterforce in the X axis direction of the object to be worn having the diameter of 15 mm was 0.19 [gf], the counterforce in the X axis direction of the object to be worn having the diameter of 17 mm was 2.22 [gf], and the counterforce in the X axis direction of the object to be worn having the diameter of 19 mm was 5.28 [gf]. In addition, the counterforce in the Z axis direction of the object to be worn having the diameter of 15 mm was 0.49 [gf], the counterforce in the Z axis direction of the object to be worn having the diameter of 17 mm was 2.13 [gf], and the counterforce in the Z axis direction of the object to be worn having the diameter of 19 mm was 3.93 [gf]. Fig. 12 shows a simulated result of a stress distribution acted on each part of the wearing part 10.

As shown in Fig. 11, the counterforce difference among the objects on which the wearing part is to be worn having different sizes can be decreased according to the embodiment as compared with the comparative embodiment. In addition, as a percentage of the change in the counterforce accompanied by the size increase of the objects to be worn is kept small, variations of a contact force with respect to the objects be worn caused by a size difference can be kept low.

As described above, according to the embodiment, since a change of the fitting property caused by the size variations of the user's fingers F can be suppressed, desirable haptic vibration can be stably presented with respect to the finger F. Alternatively, the biodata can be stably acquired from the finger F.

According to the embodiment, since the size variations of the fingers can be accommodated without requiring a size adjustment mechanism such as a buckle and a hook-and-loop fastener, wearing property or takeoff property can be enhanced. Furthermore, since it does not need to have the size variations tailored to finger thicknesses, inventory control costs can also be decreased. As further described later, the object on which the wearing part is to be worn is not limited to the finger, the present invention is applicable as the wearing part of wrists, ankles, a head, or the like, and a shape and a size of the wearing part are easily optimized by tailoring to a location to be worn.

### <Second embodiment>

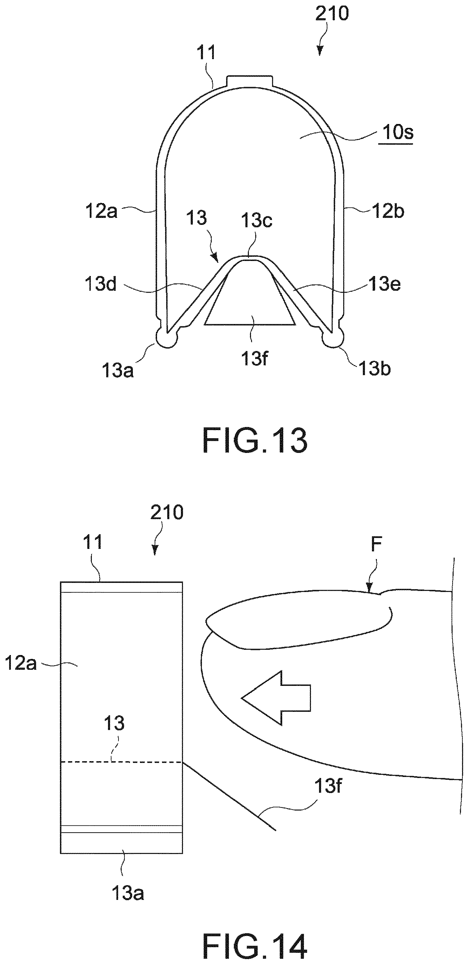

Fig. 13 and Fig. 14 are a front view and a side view showing a wearing part according to a second embodiment of the present invention.

Hereinafter, configurations different from the first embodiment will be mainly described. Configurations similar to the first embodiment are denoted by the similar reference signs, and description thereof will be omitted or simplified.

A structure of the pressing part 13 of the wearing part 210 in this embodiment is different from that in the first embodiment. Specifically, the pressing part 13 has a guide piece 13f that guides insertion of the object on which the wearing part is to be worn (finger, etc.) into the hollow part 10s in this embodiment. The guide piece 13f is formed of a tongue-like flat plate extending outwardly from an insertion side edge of the pressing part 13 and typically has a bottom-wide shape extending diagonally downward of the wearing part 210.

As described above, the hollow part 10s of the wearing part 210 in the natural status has an initial shape smaller than the size of the finger F. Thus, in a case where the pressing part 13 is provided with the guide piece 13f as in this embodiment and the finger F is disposed along an upper surface of the guide piece 13f, the finger F easily inserts the finger F into the hollow part 10s. This allows the wearing property of the wearing part 210 to be further enhanced.

The guide piece 13f is typically integrally formed with the pressing part 13 by using the same material, but may be formed of a material different from that of the pressing part 13. The guide piece 13f may be formed with a curved surface having a convex shape at a finger F side as well as a flat plate shape.

### <Third embodiment>

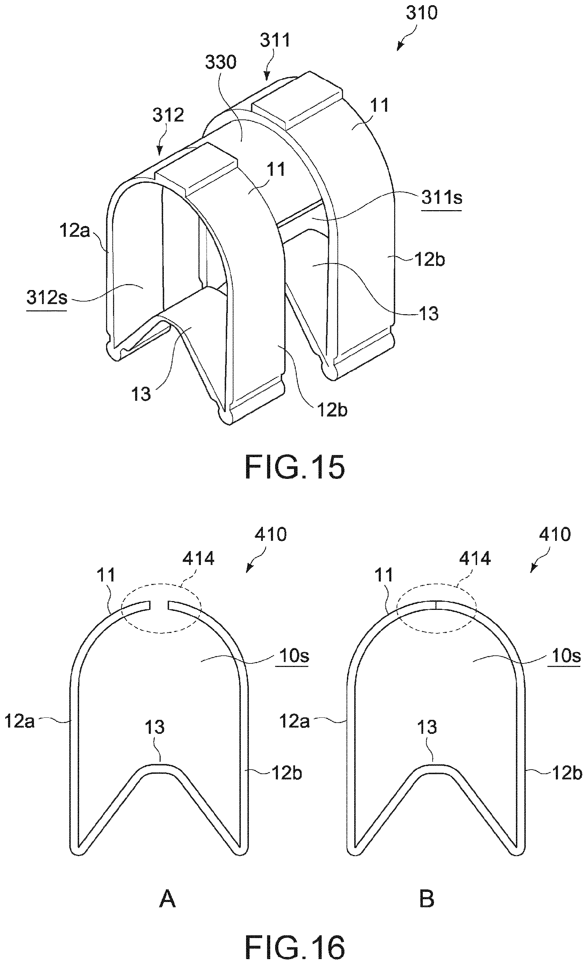

Fig. 15 is a perspective view showing a wearing part according to a third embodiment of the present invention.

Hereinafter, configurations different from the first embodiment will be mainly described. Configurations similar to the first embodiment are denoted by the similar reference signs, and description thereof will be omitted or simplified.

A wearing part 310 in this embodiment has a double structure that a first annular member 311 and a second annular member 312 are connected. The first and second annular members 311 and 312 have similar structures and each includes the base part 11, the pair of arms 12a and 12b, and the pressing part 13. The electronic devices 20 (not shown) are arranged at the pressing parts 13 of the respective annular members 311 and 312.

The first and second annular members 311 and 312 are connected each other via a connecting part 330 such that the respective hollow parts 311s and 312s are opposed each other. The connecting part 330 is arranged at the base parts 11 of the respective annular members 311 and 312 and is formed to have an appropriate size so as not to inhibit the shape change of the both annular members 311 and 312.

The first and second annular members 311 and 312 may have the same size. In this embodiment, the first and second annular members 311 and 312 may have structures in which opening areas of the hollow parts 311s and 312s may be different each other. In this embodiment, a fingertip is positioned at the second annular member 312 not the first annular member 311. Thus, the hollow part 311s has the opening area slightly larger than that of the hollow part 312s.

According to this embodiment, the electronic devices 20 are capable of being brought in contact with a plurality of locations of the finger F. In addition, the wearing part 310 is capable of being fitted to the plurality locations of the finger F with an almost uniform contact force. Also advantageously, since the hollow part 312s of the second annular member 312 in which the finger is first inserted has the opening area wider than that of the hollow part 311s of the first annular member 311, the wearing part 310 is easily worn.

A wearing position of the finger F in the wearing part 310 is not especially limited and may be the fingertip, between the first joint and the second joint of the finger, or the like, for example. The first and second annular members 311 and 312 are integrally formed each other, but may be separate parts. The number of the annular member is not limited to two (double), and may be three (triple) or more.

### <Fourth embodiment>

Fig. 16 is a front view showing a wearing part according to a fourth embodiment of the present invention.

Hereinafter, configurations different from the first embodiment will be mainly described. Configurations similar to the first embodiment are denoted by the similar reference signs, and description thereof will be omitted or simplified.

A structure of the base part 11 of the wearing part 410 in this embodiment is different from that in the first embodiment. Specifically, the wearing part 410 in this embodiment has a separating mechanism 414 capable of separating the base part 11 in the perimeter direction.

Since the pair of arms 12a and 12b have an adequate spring property, the object to be worn can wear the wearing part 410 while both sides of the base part 11 are stretched and widen left and right via the separating mechanism 414. Thus, it enhances the wearing property with respect to a human body part other than the finger such as a wrist, an ankle and a body. In addition, if the object on which the wearing part is to be worn is relatively long, it will be possible to easily wear the wearing part other than the way of insertion.

A structure of the separating mechanism 414 is not especially limited and may be a magnetic adsorption method by using magnet, for example. Also, a mechanical engagement mechanism may be used.

### <Fifth embodiment>

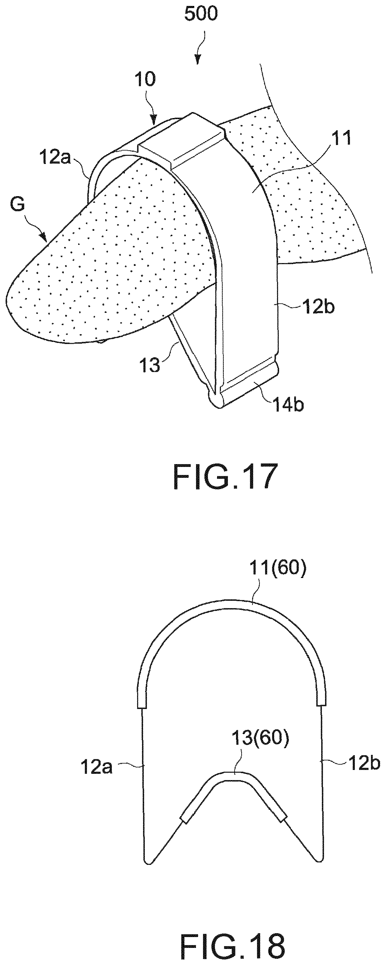

Fig. 17 is a perspective view showing a wearable apparatus according to a fifth embodiment of the present invention.

Hereinafter, configurations different from the first embodiment will be mainly described. Configurations similar to the first embodiment are denoted by the similar reference signs, and description thereof will be omitted or simplified.

A wearable apparatus 500 in this embodiment includes the wearing part 10, an electronic device (not shown), and a glove G. Note that the electronic device is arranged at the pressing part 13 (top part 13c) of the wearing part 10 similar to the first embodiment.

The glove G is formed of a glove or a fingerstall that the user's finger wears. The glove G is typically made of an elastic material such as a rubber material and a fiber material. The wearing part 10 has a structure similar to the first embodiment, is worn outside the glove G, and presents the vibration haptics to the user's finger via the glove G. The wearing part 10 is formed separately from the glove G. However, in a case where the glove G is made of a resin material, the wearing part 10 may be formed integrally with the glove G.

### <Applications>

The wearing part having the above-described structure in each embodiment or the wearable apparatus including the same have following applications.

### (Application 1)

A haptic glove according to the structure of the present technology can realize haptic experience of touching a virtual object or feeling counterforce in a game and various applications that assumes VR (Virtual Reality), AR (Augmented Reality), or the like. It is also possible to operate virtual devices. Such applications are not only simple games, but also are expected in the field to enhance capabilities in acquiring work skills, training, etc. A glove capable of presenting the haptic feedback having a good wearing property, stability, and high quality is expected.

Since most of the "works" are done by hands and fingers, the most necessary is to provide fingers and the hands with high quality haptic feedback. It should be appreciated that it is possible to provide haptic feedback shoes, a haptic feedback waist belt, and the like other than the fingers and the hand depending on the applications.

### (Application 2)

The present technology is applicable as a variety of wearing devices for acquiring biodata, for example, including a device for detecting a human weak electrical signal such as a myoelectric sensor, a device for detecting vibration on a human body surface such as a pulse wave sensor, and a device for sensing chemical information on a human skin surface such as a perspiration sensor.

### (Application 3)

The present technology is applicable to a sensor for detecting a hand movement by a resistance change material, etc. represented by a data glove, etc. By using such glove, the device can be operated with a hand gesture or the like.

### <Modification>

In the above-described embodiments, the wearing part is made of a synthetic resin material or a metal material. Alternatively, the base part 11 and pressing part 13 (top part thereof) may be made of a soft material 60 such as synthetic leather, a cloth material and a cushioning material, for example, as schematically shown in Fig. 18. Thus, a feeling of oppression upon wearing can be relaxed.

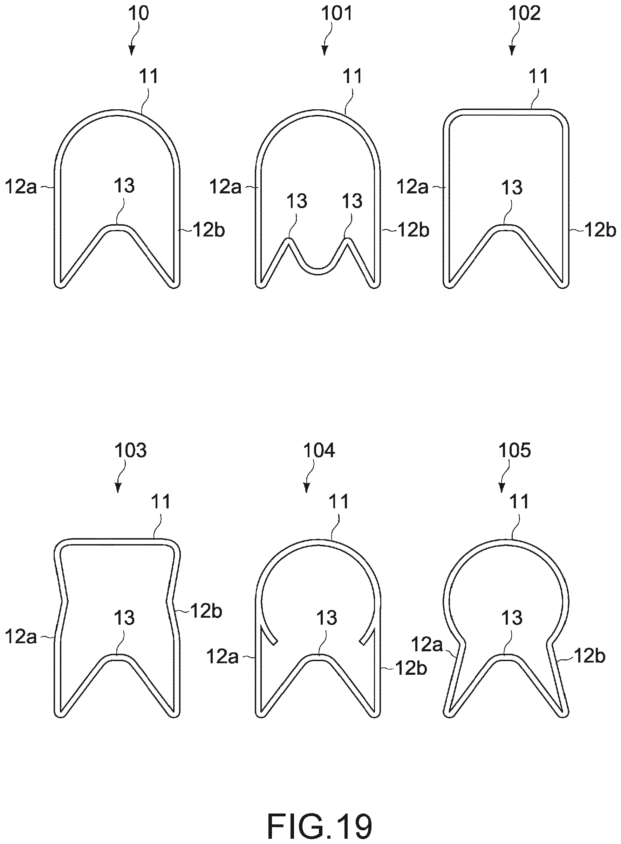

Forms of the wearing part are not limited to the above-described respective embodiments and a variety of forms are applicable as shown in Fig. 19. For example, a wearing part 101 includes a plurality of pressing parts 13. A wearing part 102 includes the base part 11 having not an arch shape but a flat shape. A wearing part 103 includes the arms 12a and 12b partly bent by internally bending both ends of the base part 11. In wearing parts 104 and 105, each of the base part 11 is formed in a circle ring-like shape.

Furthermore, in the above-descrbied embodiments, the object to be inserted is illustrated mainly taking the user's finger as an example. Other than it, the present technology is applicable to general wearing parts including other human body parts such as a wrist, a back of a hand, a palm, an arm, an ankle, a knee, a thigh, an abdomen, a chest, a neck and a head.

The object to be inserted may be other than a human. For example, the present technology is applicable to observation of a plant growth such that a plant, e.g., a planted tree, wears the wearing part and to detection of a physical quantity such as a vibration and a temperature such that a building structure such as a building support wears the wearing part.

Furthermore, the wearable apparatus may include the wearing part and the electronic device as respective separate components, and only the wearing part may be used alone.

### Reference Signs List

10, 210, 310, 410 wearing part
10s, 311s, 312s hollow part
11 base part
12a, 12 arms
13 pressing part
13a and 13b bending part
13c top part
13d and 13e linking part
13f guide piece
20 electronic device
100, 500 wearable apparatus
F, F1, F2 finger

## Claims

1. A wearing part (10), comprising:
an annular member having a hollow part (10s) into which an object on which the wearing part is to be worn is insertable, wherein
the annular member includes
a base part (11),
a pair of arms (12a, 12b) linked to both ends of the base part, and
a bending-deformable pressing part (13) that is linked to edges of the pair of arms, is opposed to the base part across the hollow part, and has a convex shape at an inner side;
**characterised in that**
the pressing part has a pair of bending parts (13a, 13b) linked to the pair of arms and a top part (13c) positioned between the pair of bending parts, and is elastically deformable in a direction in which the top part is apart from the base part depending on a distance increase between the pair of arms.

2. A wearable apparatus (100), comprising:
the wearable part (10) according to claim 1 and
an electronic device (20) arranged at at least one of the base part or the pressing part and capable of being in contact with the object on which the wearing part is to be worn inserted into the hollow part.

3. The wearable apparatus according to claim 2, wherein
the pressing part further includes a pair of link parts (13d, 13e) that link the pair of bending parts to the top part, and an angle between the linked parts is variable depending on a distance change between the pair of arms.

4. The wearable apparatus according to claim 2, wherein
the base part has an arch shape capable of being in contact with an outer perimeter surface of the object on which the wearing part is to be worn inserted into the hollow part.

5. The wearable apparatus according to claim 4, wherein
the base part has a pair of low rigid parts (11a, 11b) linked to the pair of arms and a high rigid part (11c) positioned between the pair of low rigid parts and having rigidity higher than the pair of low rigid parts.

6. The wearable apparatus according to claim 5, wherein
the pair of arms have rigidity higher than the pair of low rigid parts.

7. The wearable apparatus according to claim 2, wherein
the pressing part has a guide piece (13f) that guides insertion of the object on which the wearing part is to be worn into the hollow part.

8. The wearable apparatus according to claim 2, wherein
the wearing part (310) has a structure of a plurality of annular members (311, 312) with hollow parts opposed to each other, and each of the plurality of annular members includes a base part, a pair of arms, and a pressing part.

9. The wearable apparatus according to claim 8, wherein
the hollow parts of the plurality of annular members have different opening areas to each other.

10. The wearable apparatus according to claim 2, wherein
the wearing part (410) further includes a separating mechanism (414) capable of separating both sides of the base part in a perimeter direction.

11. The wearable apparatus according to claim 2, wherein
the wearing part is made of a synthetic resin material or a metal material.

12. The wearable apparatus according to claim 2, wherein
the electronic device includes a haptic feedback device.

13. The wearable apparatus according to claim 2, wherein
the electronic device includes a sensor device.

## Patentansprüche

1. Am Körper getragenes Teil (10), umfassend:
ein ringförmiges Element mit einem hohlen Teil (10s), in das ein Objekt, an dem das Trageteil zu tragen ist, einführbar ist, wobei
das ringförmige Element umfasst:
ein Basisteil (11),
ein Paar von Armen (12a, 12b), die mit beiden Enden des Basisteils verbunden sind, und
ein biegeverformbares Druckteil (13), das mit den Kanten des Paars von Armen verbunden ist, dem Basisteil über den hohlen Teil gegenüberliegt und an einer Innenseite eine konvexe Form aufweist;
**dadurch gekennzeichnet, dass**
das Druckteil ein Paar von Biegeteilen (13a, 13b), die mit dem Paar von Armen verbunden sind, und einen oberen Teil (13c) aufweist, der zwischen dem Paar von Biegeteilen angeordnet ist und in eine Richtung elastisch verformbar ist, in der der obere Teil abhängig von einer Abstandszunahme zwischen dem Paar von Armen von dem Basisteil beabstandet ist.

2. Am Körper tragbare Vorrichtung (100), umfassend:
das tragbare Teil (10) gemäß Anspruch 1 und eine elektronische Vorrichtung (20), die an mindestens einem des Basisteils oder des Druckteils angeordnet ist und mit dem in den Hohlraum eingeführten Objekt in Kontakt gebracht werden kann, an dem das tragbare Teil zu tragen ist.

3. Am Körper tragbare Vorrichtung gemäß Anspruch 2, wobei
das Druckteil ferner ein Paar von Verbindungsteilen (13d, 13e) umfasst, die das Paar von Biegeteilen mit dem oberen Teil verbinden, und ein Winkel zwischen den verbundenen Teilen abhängig von einer Abstandsänderung zwischen dem Paar von Armen variabel ist.

4. Am Körper tragbare Vorrichtung gemäß Anspruch 2, wobei
das Basisteil eine Bogenform aufweist, die mit einer äußeren Umfangsfläche des in den hohlen Teil eingeführten Objekts, an dem das Trageteil zu tragen ist, in Kontakt gebracht werden kann.

5. Am Körper tragbare Vorrichtung gemäß Anspruch 4, wobei
das Basisteil ein Paar niedrig-steifer Teile (11a, 11b), die mit dem Paar von Armen verbunden sind, und ein hoch-steifes Teil (11c) aufweist, das zwischen dem Paar niedrig-steifer Teile angeordnet ist und eine höhere Steifigkeit als das Paar niedrig-steifer Teile aufweist.

6. Am Körper tragbare Vorrichtung gemäß Anspruch 5, wobei
das Paar von Armen eine höhere Steifigkeit als das Paar niedrig-steifer Teile aufweist.

7. Am Körper tragbare Vorrichtung gemäß Anspruch 2, wobei
das Druckteil ein Führungsstück (13f) aufweist, das das Einführen des Objekts, an dem das Trageteil zu tragen ist, in den hohlen Teil führt.

8. Am Körper tragbare Vorrichtung gemäß Anspruch 2, wobei
das Trageteil (310) eine Struktur aus einer Vielzahl von ringförmigen Elementen (311, 312) mit einander gegenüberliegenden hohlen Teilen aufweist und jedes der Vielzahl von ringförmigen Elementen ein Basisteil, ein Paar von Armen und ein Druckteil umfasst.

9. Am Körper tragbare Vorrichtung gemäß Anspruch 8, wobei
die hohlen Teile der Vielzahl von ringförmigen Elementen untereinander unterschiedliche Öffnungsbereiche aufweisen.

10. Am Körper tragbare Vorrichtung gemäß Anspruch 2, wobei
das Trageteil (410) ferner einen Trennmechanismus (414) umfasst, der in der Lage ist, beide Seiten des Basisteils in einer Umfangsrichtung zu trennen.

11. Am Körper tragbare Vorrichtung gemäß Anspruch 2, wobei
das Trageteil aus einem Kunstharzmaterial oder einem metallischen Material besteht.

12. Am Körper tragbare Vorrichtung gemäß Anspruch 2, wobei
die elektronische Vorrichtung eine haptische Rückmeldevorrichtung aufweist.

13. Am Körper tragbare Vorrichtung gemäß Anspruch 2, wobei
die elektronische Vorrichtung eine Sensorvorrichtung aufweist.

## Revendications

1. Élément à porter sur soi (10), comprenant :
un organe annulaire pourvu d'un élément creux (10s) dans lequel peut s'insérer un objet sur lequel l'élément à porter sur soi est destiné à être porté sur soi,
l'organe annulaire comportant
un élément de base (11),
une paire de bras (12a, 12b) reliés aux deux extrémités de l'élément de base, et
un élément de pression déformable par flexion (13) qui est relié à des bords de la paire de bras, qui est opposé à l'élément de base à l'autre bout de l'élément creux, et qui est pourvu d'une forme convexe au niveau d'un côté intérieur ;
**caractérisé en ce que**
l'élément de pression est pourvu d'une paire d'éléments de flexion (13a, 13b) reliés à la paire de bras et d'un élément supérieur (13c) positionné entre la paire d'éléments de flexion, et est déformable élastiquement dans une direction dans laquelle l'élément supérieur est séparé de l'élément de base en fonction d'une augmentation de distance entre la paire de bras.

2. Appareil à porter sur soi (100), comprenant :
l'élément à porter sur soi (10) selon la revendication 1, et
un dispositif électronique (20) agencé au niveau de l'élément de base et/ou de l'élément de pression et susceptible de venir au contact de l'objet sur lequel l'élément à porter sur soi est destiné à être porté sur soi, inséré dans l'élément creux.

3. Appareil à porter sur soi selon la revendication 2, dans lequel
l'élément de pression comporte en outre une paire d'éléments de liaison (13d, 13e) qui relient la paire d'éléments de flexion à l'élément supérieur, et un angle entre les éléments reliés est variable en fonction d'une variation de distance entre la paire de bras.

4. Appareil à porter sur soi selon la revendication 2, dans lequel
l'élément de base est pourvu d'une forme arquée susceptible de venir au contact d'une surface circonférentielle extérieure de l'objet sur lequel l'élément à porter sur soi est destiné à être porté sur soi, inséré dans l'élément creux.

5. Appareil à porter sur soi selon la revendication 4, dans lequel
l'élément de base est pourvu d'une paire d'éléments de faible rigidité (11a, 11b) reliés à la paire de bras et d'un élément de haute rigidité (11c) positionné entre la paire d'éléments de faible rigidité et pourvu d'une rigidité supérieure à celle de la paire d'éléments de faible rigidité.

6. Appareil à porter sur soi selon la revendication 5, dans lequel
la paire de bras est pourvue d'une rigidité supérieure à celle de la paire d'éléments de faible rigidité.

7. Appareil à porter sur soi selon la revendication 2, dans lequel
l'élément de pression est pourvu d'une pièce de guidage (13f) qui guide l'insertion de l'objet, sur lequel l'élément à porter sur soi est destiné à être porté sur soi, dans l'élément creux.

8. Appareil à porter sur soi selon la revendication 2, dans lequel
l'élément à porter sur soi (310) est pourvu d'une structure composée d'une pluralité d'organes annulaires (311, 312) dotés d'éléments creux opposés entre eux, et
chacun de la pluralité d'organes annulaires comporte un élément de base, une paire de bras et un élément de pression.

9. Appareil à porter sur soi selon la revendication 8, dans lequel
les éléments creux de la pluralité d'organes annulaires sont pourvus de zones d'ouverture différentes entre elles.

10. Appareil à porter sur soi selon la revendication 2, dans lequel
l'élément à porter sur soi (410) comporte en outre un mécanisme de séparation (414) susceptible de séparer les deux côtés de l'élément de base dans une direction circonférentielle.

11. Appareil à porter sur soi selon la revendication 2, dans lequel
l'élément à porter sur soi se compose d'un matériau en résine synthétique ou d'un matériau métallique.

12. Appareil à porter sur soi selon la revendication 2, dans lequel
le dispositif électronique comporte un dispositif de retour haptique.

13. Appareil à porter sur soi selon la revendication 2, dans lequel
le dispositif électronique comporte un dispositif capteur.
